# EUROPÄISCHE PATENTSCHRIFT

(11) **EP 0 918 792 B1**
(45) Veröffentlichungstag und Bekanntmachung des Hinweises auf die Patenterteilung: **27.09.2000**
(21) Anmeldenummer: 97934552.7
(22) Anmeldetag: 29.07.1997
(51) Int. Cl.: C07K 5/02, C07D 207/22, C07D 211/78, A61K 38/05

(54) **DIPEPTIDISCHE BENZAMIDINE ALS KININOGENASEN-INHIBITOREN**
DIPEPTIDE BENZAMIDINE AS A KININOGENASE INHIBITOR
BENZAMIDINES DIPEPTIDIQUES UTILISEES COMME INHIBITEURS DE LA KININOGENASE

(30) Priorität: 14.08.1996 DE 19632772
(43) Veröffentlichungstag der Anmeldung: 02.06.1999
(73) Patentinhaber: BASF AKTIENGESELLSCHAFT, 67056 Ludwigshafen (DE)
(72) Erfinder: BAUCKE, Dorit, D-68163 Mannheim (DE); LANGE, Udo, D-67063 Ludwigshafen (DE); MACK, Helmut, D-67067 Ludwigshafen (DE); PFEIFFER, Thomas, D-67459 Böhl-Iggelheim (DE); SEITZ, Werner, D-68723 Plankstadt (DE); ZIERKE, Thomas, D-67459 Böhl-Iggelheim (DE); HÖFFKEN, Hans, Wolfgang, D-67069 Ludwigshafen (DE); HORNBERGER, Wilfried, D-67434 Neustadt (DE)
(86) Internationale Anmeldenummer: EP9704105
(87) Internationale Veröffentlichungsnummer: WO9806740

(56) Entgegenhaltungen:
- WO-A-94/29336
- WO-A-96/25426

## Beschreibung

Die vorliegende Erfindung betrifft neue Benzamidine, ihre Herstellung und ihre Verwendung als kompetitive Inhibitoren von Trypsin-ähnlichen Serinproteasen, besonders Thrombin und Kininogenasen wie Kallikrein. Die Erfindung bezieht sich auch auf pharmazeutische Zusammensetzungen, die die Verbindungen als aktive Bestandteile enthalten, sowie die Verwendung der Verbindungen als Thrombininhibitoren, Antikoagulantien und als antiinflammatorische Agenzien.

Thrombin gehört zur Gruppe der Serinproteasen und spielt als terminales Enzym in der Blutgerinnungskaskade eine zentrale Rolle. Sowohl die intrinsische als auch die extrinsische Gerinnungskaskade führen über mehrere Verstärkungsstufen zur Entstehung von Thrombin aus Prothrombin. Die thrombinkatalysierte Spaltung von Fibrinogen zu Fibrin leitet dann die Blutgerinnung und die Aggregation der Thrombozyten ein, die ihrerseits durch die Bindung von Plättchenfaktor 3 und Gerinnungsfaktor XIII sowie eine ganze Reihe von hochaktiven Mediatoren die Thrombinbildung verstärken.

Thrombinbildung und -wirkung sind zentrale Ereignisse bei der Entstehung sowohl von weißen, arteriellen als auch von roten, venösen Thromben und daher potentiell wirksame Angriffspunkte für Pharmaka. Thrombininhibitoren sind im Gegensatz zu Heparin in der Lage, unabhängig von Kofaktoren gleichzeitig die Wirkungen von freiem Thrombin als auch an Thrombozyten gebundenes vollständig zu hemmen. Sie können in der Akutphase thromboembolische Ereignisse nach perkutaner transluminaler koronarer Angioplastie (PTCA) und Lyse verhindern und als Antikoagulantien in der extrakorporalen Zirkulation (Herz-Lungen-Maschine, Hämodialyse) dienen. Sie können auch allgemein zur Thromboseprophylaxe, beispielsweise nach chirurgischen Eingriffen dienen.

Es ist bekannt, daß synthetische Argininderivate die Enzymaktivität des Thrombins beeinflussen, indem sie mit dem aktiven Serinrest der Protease Thrombin in Wechselwirkung treten. Peptide auf der Basis Phe-Pro-Arg, in denen die N-terminale Aminosäure in der D-Form vorliegt, haben sich als besonders günstig erwiesen. D-Phe-Pro-Arg-isopropylester ist als kompetitiv wirkender Thrombininhibitor beschrieben (C.Mattson u.a., Folia Haematol, 109, 43 bis 51, 1983).

Die Derivatisierung des C-Terminus Arginin zum Aldehyd führt zu einer Verstärkung der Inhibitorwirkung. So sind eine Vielzahl von Arginalen beschrieben, die die Hydroxylgruppe des "aktiven" Serins halbacetalisch zu binden vermögen (EP 185390, 479489, 526877, 542525; WO 93/15756, 93/18060).

Die thrombininhibitorische Wirksamkeit peptidischer Ketone, fluorierter Alkylketone, sowie von Ketoestern, Borsäurederivaten, Phosphorsäureestern und a-Ketocarbonsäureamiden ist ebenfalls mit dieser Serin-Wechselwirkung erklärbar (EP 118280, 195212, 362002 364344, 410411, 471651, 589741, 293881, 503203, 504064, 530167; WO 92/07869, 94/08941).

Bei den von J. Oleksyszyn u.a. in J. Med. Chem. 37, 226 bis 231 (1994) beschriebenen peptidischen 4-Amidinophenyl-glycinphosphonat-diphenylestern handelt es sich um irreversible Thrombininhibitoren mit unzureichender Selektivität gegenüber anderen Serinproteasen.

In DE 3 108 810, WO 93/11152 und EP 601 459 sind Agmatin und damit Arginin-Derivate beschrieben, die keine Wechselwirkung mit dem aktiven Serin der Serinproteasen eingehen können.

WO 94/29336, EP 0 601 459 und WO 95/23609 stellen eine Weiterentwicklung dar, wobei der Agmatin- durch einen Arylamidinrest ersetzt ist.

Kininogenasen sind Serinproteasen, die aus Kininogenen vasoaktive Peptide, die sog. Kinine (Bradykinin, Kallidin und Met-Lysbradylinin), freisetzen. Kininogene stellen multifunktionale Proteine dar, die in Kaskadenreaktionen der Gerinnung und Entzündung auftreten. Als Inhibitoren schützen sie Zellen vor der Zerstörung durch Cystein-Proteasen (Müller Esterl, 1985, FEBS Lett. 182, 310-314).

Wichtige Kininogenasen sind Plasma-Kallikrein, Gewebs-Kallikrein und Mastzellen-Tryptase.

Kinine wie Bradykinin und Kallidin sind vasoaktive Peptide, die eine Vielzahl biologischer Prozesse beeinflussen. Sie spielen in entzündlichen Prozessen eine wesentliche Rolle. Durch Erhöhung der vaskulären Permeabilität führen sie zu Hypotension und Ödemen. Weiterhin sind sie sehr potente schmerzproduzierende Autacoide und haben als zelluläre Mediatoren in der Pathophysiologie des Asthmas, der allergischen Rhinitis und der Arthritis große Bedeutung (K.D. Bhoola, C.D. Figueroa, K. Worthy, Pharmacological Reviews 1992, 44 (1), 1-80).

Unabhängig von den Mechanismen, die entzündlichen Prozessen zugrundeliegen, kommt es zum Austritt von Flüssigkeit aus den Blutgefäßen, die alle Protein-Systeme des zirkulierenden Blutes enthält. Das bedeutet, daß der Austritt von Plasmaflüssigkeit aus den Gefäßen in Krankheiten wie Asthma, Rhinitis und entzündungsbedingten inneren Krankheiten eine Rolle spielt. Besonders in allergischen Prozessen wird dabei Mastzell-Tryptase freigesetzt (Salomonsson et al., Am. Rev. Respir. Dis., 1992, 146, 1535-1542).

Die Arginin-Chloromethylketone H-(D)-Pro-Phe-Arg-CH₂Cl und H-(D)-Phe-Phe-Arg-CH2-Cl wurden von Kettner und Shaw als Plasma-Kallikreininhibitoren beschrieben (Biochem. 1978, 17, 4778-4784 und Meth. Enzym. 1981, 80, 826-842).

Verschiedene synthetische Derivate von Benzamidinen und Benzylaminen erwiesen sich als Inhibitoren von Plasmakallikrein, wobei die Benzamidine eine wesentlich stärkere inhibitorische Wirkung aufweisen (F. Markward, S. Drawert, P. Walsmann, Biochemical Pharmacology 1974, 23, 2247-2256).

Auch PKSI-527, das Hydrochlorid von N-(trans-4-aminomethylcyclohexylcarbonyl)-L-phenylalanin-4-carboxymethyl-anilid, ist ein wirksamer Inhibitor für diese Kininogenase (Wanaka, Ohamoto et al., Thromb. Res. 1990, 57 (6), 889-895).

Gegenstand der Erfindung sind Verbindungen der Formel I 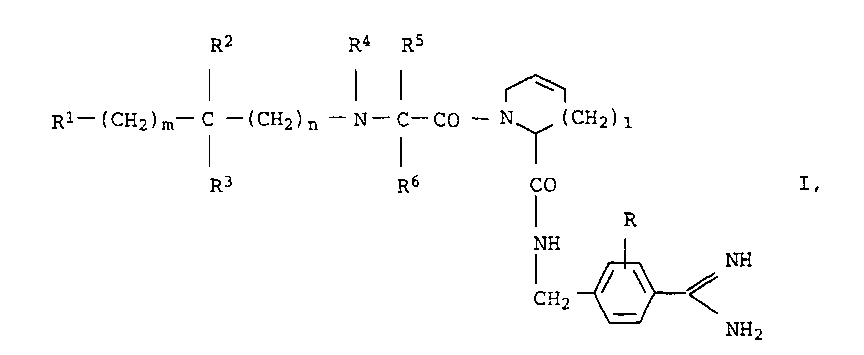 worin die Reste R, R¹, R², R³, R⁴, R⁵ und R⁶ sowie l, m und n folgende Bedeutungen besitzen:
- l: 0 oder 1,
- m: 0,1 oder 2,
- n: 0,1 oder 2,
- R: H oder C₁₋₄-Alkyl-,
- R¹: HOOC-, C₁₋₆-Alkyl-OOC-, Benzyl-OOC- oder -OH,
- R²: H-, C₁₋₄-Alkyl- oder R¹-(CH₂)ₘ-,
- R³: H- oder C₁₋₄-Alkyl-, welches durch -OH oder -COOH substituiert sein kann,
- R⁴: H-, C₁₋₄-Alkyl-, HOOC-C₁₋₄-alkylen-,
- R⁵: C₁₋₈-Alkyl-, Cycloalkyl-(CR⁸R⁹)ᵣ-, (r = 0 oder 1, R⁸, R⁹ = H-, Cycloalkyl- oder C₁₋₄-Alkyl-), worin bis zu vier CH₂-Gruppen des Cycloalkylrestes unabhängig voneinander durch CR¹⁰R¹¹ (R¹⁰ = H-oder C₁₋₄-Alkyl-, R¹¹ = C₁₋₄-Alkyl-) und/oder die an CR⁸R⁹ gebundene CH-Gruppe des Cycloalkylrestes durch CR¹² (R¹² = C₁₋₄-Alkyl-) ersetzt sein können und/oder eine oder zwei C-C-Einfachbindung(en) im Ring durch eine C=C-Doppelbindung ersetzt sein können,
- R⁶: H-, C₁₋₄-Alkyl- oder
- R⁴ und R⁵: zusammen -CH₂-CH₂-CH(R⁷)-, (R⁷ = H-, Phenyl- oder Cyclohexyl-)
- R² und R⁵: zusammen -CH₂-CH₂- oder -CH₂-CH₂-CH₂-, worin ein Wasserstoffatom durch C₁₋₄-Alkyl-, Phenyl- oder Cycloalkyl- ersetzt sein kann,
sowie deren Salze mit physiologisch verträglichen Säuren.

Die durch -NR⁴-C(R⁵R⁶)-CO-dargestellten Aminosäurereste sind vorzugsweise (D)-konfiguriert, das 3,4-Dehydroprolin bzw. die 4,5-Dehydropipecolinsäure vorzugsweise (L)-konfiguriert.

Bevorzugt sind Verbindungen der Formel I, worin die Gruppe 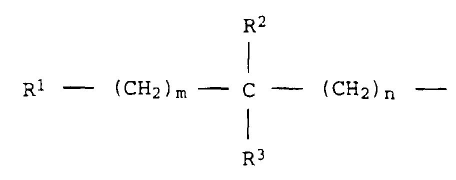 HOOC-(CH₂)ₜ- (t = 1, 2 oder 3), (HOOC-CH₂)₂-CH-, (HO-CH₂)₂CH-, HOOC-CH₂-CH(COOH)-, HOOC-CH(CH₂-CH₂-OH)-, HOOC-CH(C₁₋₄-Alkyl)-, C₁₋₄-Alkyl-OOC-CH₂-, Benzyl-OOC-CH₂- ist,
und worin die Gruppe 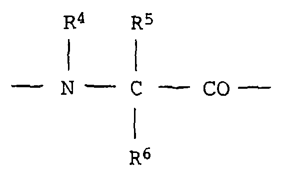 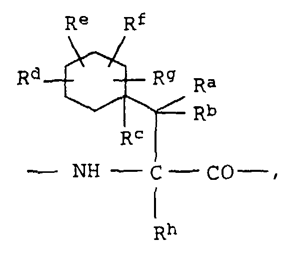 (R^{a}, R^{b} = H, Cyclohexyl- oder C₁₋₄-Alkyl-) (R^{c}, R^{d}, R^{e}, R^{f}, R^{g}, R^{h} = H- oder C₁₋₄-Alkyl-, wobei die CH₂-Gruppen des Ringes einfach oder zweifach substituiert sein können), 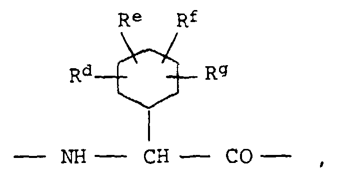 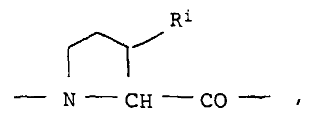 (Rⁱ) = Phenyl- oder Cyclohexyl-) 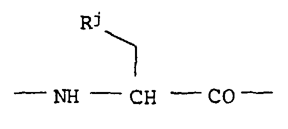
- R^{j} =: Cyclopentyl-, Cycloheptyl-, 1-Adamantyl-, 1-Norbornyl-, 1-Bicyclo[2.2.2]octyl-, Neopentyl-, tert.-Butyl-, Diisopropylmethyl- oder 1-(1,4-Cyclohexadienyl-))
bedeutet, wobei dieser Baustein vorzugsweise D-konfiguriert ist,
- l: 0 ist und
- R: H- oder CH₃- ist.

Bevorzugt sind weiter Verbindungen der Formel I, worin die Gruppe 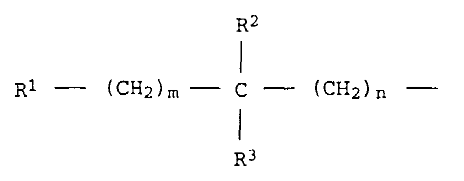 HOOC-(CH₂)ₜ- (t = 1, 2 oder 3), (HOOC-CH₂)₂-CH-, (HO-CH₂)₂CH-, HOOC-CH₂-CH(COOH)-, HOOC-CH(CH₂-CH₂-OH)-, HOOC-CH(C₁₋₄-Alkyl)-, C₁₋₄-Alkyl-OOC-CH₂-, Benzyl-OOC-CH₂- ist,
und worin die Gruppe 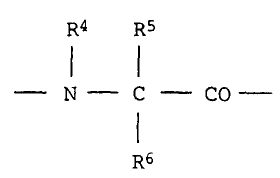 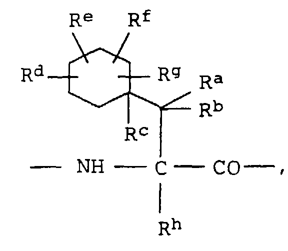 (R^{a}, R^{b} = H, Cyclohexyl- oder C₁₋₄-Alkyl-) (R^{c}, R^{d}, R^{e}, R^{f}, R^{g}, R^{h} = H- oder C₁₋₄-Alkyl-, wobei die CH₂-Gruppen des Ringes einfach oder zweifach substituiert sein können), 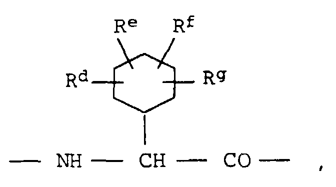 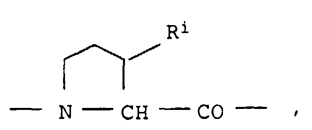 (Rⁱ) = Phenyl- oder Cyclohexyl-) 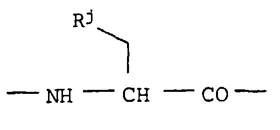
- R^{j} =: Cyclopentyl-, Cycloheptyl-, 1-Adamantyl-, 1-Norbornyl-, 1-Bicyclo[2.2.2] octyl-, Neopentyl-, tert.-Butyl-, Diisopropylmethyl- oder 1-(1,4-Cyclohexadienyl-))
bedeutet, wobei dieser Baustein vorzugsweise D-konfiguriert ist,
- l: 1 ist und
- R: H- oder CH₃-ist.

Bevorzugt sind auch Serinprotease-Inhibitoren mit dem strukturellen Element der Formel 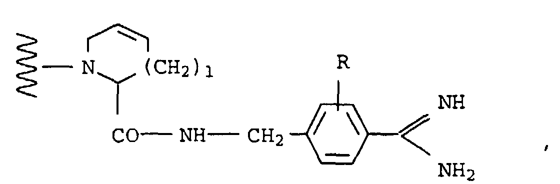 worin l = 0 oder 1 und R = H oder C₁-C₄-Alkyl, insbesonder CH₃, bedeutet.

Als Zwischenverbindungen sind Verbindungen der Formel II bevorzugt 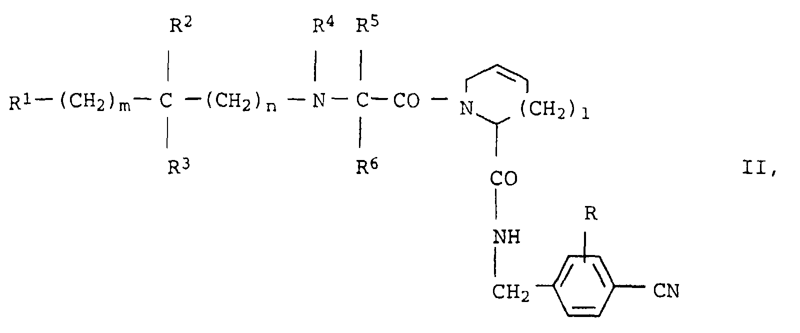 worin die Gruppe 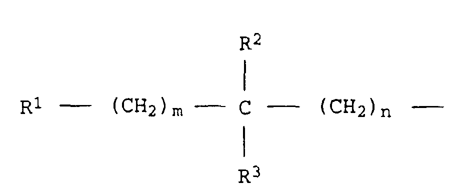 HOOC-(CH₂)ₜ- (t = 1, 2 oder 3), (HOOC-CH₂)₂-CH-, (HO-CH₂)₂CH-, HOOC-CH₂-CH(COOH)-, HOOC-CH(CH₂-CH₂-OH)-, HOOC-CH(C₁₋₄-Alkyl)-, C₁₋₄-Alkyl-OOC-CH₂-, Benzyl-OOC-CH₂- ist,
und worin die Gruppe 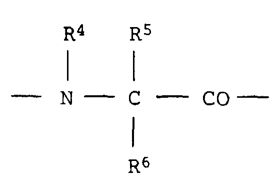 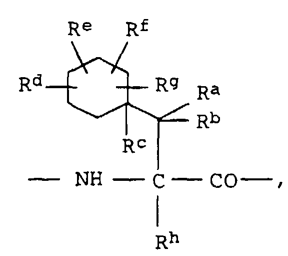 (R^{a}, R^{b} = H, Cyclohexyl- oder C₁₋₄-Alkyl-) (R^{c}, R^{d}, R^{e}, R^{f}, R^{g}, R^{h} = H- oder C₁₋₄-Alkyl-, wobei die CH₂-Gruppen des Ringes einfach oder zweifach substituiert sein können), 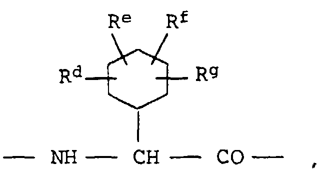 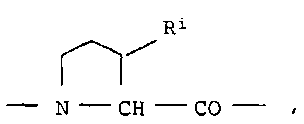 (Rⁱ) = Phenyl- oder Cyclohexyl-) 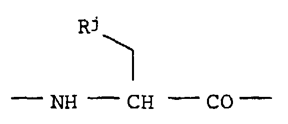
- R^{j} =: Cyclopentyl-, Cycloheptyl-, 1-Adamantyl-, 1-Norbornyl-, 1-Bicyclo[2.2.2]octyl-, Neopentyl-, tert.-Butyl-, Diisopropylmethyl- oder 1-(1,4-Cyclohexadienyl-))
bedeutet, wobei dieser Baustein vorzugsweise D-konfiguriert ist,
- l: 0 oder 1 ist und
- R: H- oder CH₃- ist.

Weiterhin sind als Zwischenstufen die Verbindungen der Formel 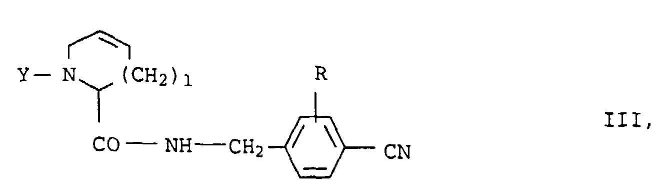 interessant, worin l und R die Bedeutung gemäß Anspruch 1 besitzt und Y eine N-Schutzgruppe, eine N-tertinal geschützte oder ungeschützte Aminosäure oder H- bedeutet.

Bevorzugte Verbindungen der Formel III sind diejenigen, in denen
- Y: Boc-, Boc-Cha-, H-Cha-, Boc-Chg-, H-Chg oder H,
- l: = 0 oder 1 und R = H oder CH3 bedeuten.

Folgende Substanzen sind besonders bevorzugt:
1. HOOC-CH₂-(D)-Cha-Pyr-NH-4-amb
2. HOOC-(CH₂)₂-(D)-Cha-Pyr-NH-4-amb
3. (HOOC-CH₂)₂CH- (D) -Cha-Pyr-NH-4-amb
4. (HO-CH₂)₂CH-(D)-Cha-Pyr-NH-4-amb
5. HOOC-CH₂-CH(COOH)-(D)-Cha-Pyr-NH-4-amb
6. HOOC-CH₂-(D)-Chg-Pyr-NH-4-amb
7. HOOC-CH₂-(D)-(α-Me)Cha-Pyr-NH-4-amb
8. HOOC-CH₂-(D,L)-(1-Me)Cha-Pyr-NH-4-amb
9. HOOC-CH₂-(D,L)-(β,β-Me₂)Cha-Pyr-NH-4-amb
10. HOOC-CH₂-(D,L)-(trans 4-Me)Cha-Pyr-NH-4-amb
11. HOOC-CH₂-(D,L)-Cycloheptylalanin-Pyr-NH-4-amb
12. HOOC-CH₂-(D,L)-1-Adamantylalanin-Pyr-NH-4-amb
13. HOOC-CH₂-(D,L)-2-Norbornylglycin-Pyr-NH-4-amb
14. HOOC-CH₂-(D,L)-(3,3-Me₂)Cha-Pyr-NH-4-amb
15. HOOC-CH₂-(D)-tert.-Butylalanin-Pyr-NH-4-amb
16. HOOC-CH₂-(D,L)(1,4-Cyclohexadien-1-yl)alanin-Pyr-NH-4-amb
17. HOOC-CH₂-(D)-Cha-Dep-NH-4-amb
18. HOOC-CH₂-(D)-Chg-Dep-NH-4-amb
19. HOOC-CH₂-(D,L)-Dch-Pyr-NH-4-amb

Hier wie auch in den Beispielen werden folgende Abkürzungen verwendet:
- amb =: amidinobenzyl
- Boc =: tert.-Butyloxycarbonyl
- Cha =: Cyclohexylalanin
- Chea =: Cycloheptylalanin
- Chg =: Cyclohexylglycin
- Dch =: Dicyclohexylalanin
- Dpa =: Diphenylalanin
- Me =: Methyl
- Pyr =: 3,4-Dehydroprolin
- Dep =: 4,5-Dehydropipecolinsäure

Für den Fall, daß -NR⁴-CR⁵R⁶-CO- ein Cyclohexylalanin-Rest ist, wurden die einzelnen C-Atome wie folgt bezeichnet:

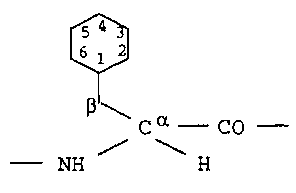

Die Verbindungen der Formel I können als solche oder in Form ihrer Salze mit physiologisch verträglichen Säuren vorliegen. Beispiele für solche Säuren sind: Salzsäure, Zitronensäure, Weinsäure, Milchsäure, Phosphorsäure, Methansulfonsäure, Essigsäure, Ameisensäure, Maleinsäure, Fumarsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin.

Die neuen Verbindungen der Formel I lassen sich bei folgenden Indikationen einsetzen:
- Krankheiten, deren Pathomechanismus direkt oder indirekt auf der proteolytischen Wirkung von Thrombin beruht,
- Krankheiten, deren Pathomechanismus auf der thrombinabhängigen Aktivierung von Rezeptoren und Signaltransduktionen beruht,
- Krankheiten, die mit Stimulation [z.B. durch PAI-1, PDGF (platelet derived growth factor), P-Selectin, ICAM-1, Tissue Factor] oder Inhibition (z.B. NO-Synthese in Glattmuskelzellen) von Genexpressionen in Körperzellen einhergehen,
- Krankheiten, die auf der mitogenen Wirkung von Thrombin beruhen,
- Krankheiten, die auf einer thrombinabhängigen Kontraktilitäts- und Permeabilitätsveränderung von Epithelzellen (z.B. Gefäßendothelzellen) beruhen,
- thrombinabhängige, thromboembolische Ereignisse wie tiefe Venenthrombose, Lungenembolie, Myocard- oder Cerebralinfarkt, Vorhofflimmern, Bypassverschluß,
- disseminierte intravasale Koagulation (DIC),
- Reokklusion und zur Verkürzung der Reperfusionszeit bei Komedikation mit Thrombolytika wie Streptokinase, Urokinase, Prourokinase, t-PA, APSAC, Plasminogenaktivatoren aus den Speicheldrüsen von Tieren sowie die rekombinanten und mutierten Formen all dieser Substanzen,
- das Auftreten von früher Reokklusion und später Restenosierung nach PTCA,
- die thrombinabhängige Proliferation von Glattmuskelzellen,
- die Akkumulation aktiven Thrombins im ZNS (z.B. bei M. Alzheimer),
- das Tumorwachstum sowie gegen die Adhäsion und Metastasierung von Tumorzellen.

Insbesondere lassen sich die neuen Verbindungen zur Therapie und Prophylaxe von thrombinabhängigen thromboembolischen Ereignissen wie tiefen Venenthrombosen, Lungenembolien, Myocard- oder Cerebralinfarkten und instabiler Angina, weiterhin zur Therapie der Disseminierten Intravasalen Koagulation (DIC) einsetzen. Weiter eignen sie sich zur Kombinationstherapie mit Thrombolytika wie Streptokinase, Urokinase, Prourokinase, t-PA, APSAC und anderen Plasminogenaktivatoren zur Verkürzung der Reperfusionszeit und Verlängerung der Reokklusionszeit.

Weitere bevorzugte Anwendungsgebiete sind die Verhinderung thrombinabhängiger früher Reokklusion und später Restenosierung nach perkutaner transluminaler koronarer Angioplasie, die Verhinderung thrombininduzierter Proliferation glatter Muskelzellen, die Verhinderung der Akkumulation aktiven Thrombins im ZNS (z.B. bei M. Alzheimer), die Tumorbekämpfung und die Verhinderung von Mechanismen, die zu Adhäsion und Metastasierung von Tumorzellen führen.

Die neuen Verbindungen lassen sich auch zur Beschichtung von künstlichen Oberflächen wie Hämodialysemembranen und den dazu erforderlichen Schlauchsystemen und Leitungen sowie von Oxygenatoren der extravasalen Zirkulation, Stents und Herzklappen verwenden.

Die neuen Verbindungen lassen sich weiter bei Krankheiten einsetzen, deren Pathomechanismus direkt oder indirekt auf der proteolytischen Wirkung von Kininogenasen, insbesondere Kallikrein beruht z.B. bei Entzündungskrankheiten wie Asthma, Pankreatitis, Rhinitis, Arthritis, Urticaria und anderen inneren Entzündungskrankheiten.

Der besondere Vorteil der neuen Verbindungen liegt darin, daß sie durch Austausch von Prolin gegen 3,4-Dehydroprolin und durch Austausch von Pipecolinsäure gegen 4,5-Dehydropipecolinsäure eine verbesserte pharmakologische Wirkung zeigen und sich daher von den in WO 94/29336 beschriebenen Verbindungen hervorheben.

Die erfindungsgemäßen Verbindungen können in üblicher Weise oral oder parenteral (subkutan, intravenös, intramuskulär, intraperitoneal, rektal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis pro Person zwischen etwa 10 und 2000 mg bei oraler Gabe und zwischen etwa 1 und 200 mg bei parenteraler Gabe. Diese Dosis kann in 2 bis 4 Einzeldosen oder einmalig am Tag als Depotform gegeben werden.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

### Experimenteller Teil

Die Verbindungen der Formel I lassen sich entsprechend Schemata I-III darstellen, wobei
A für 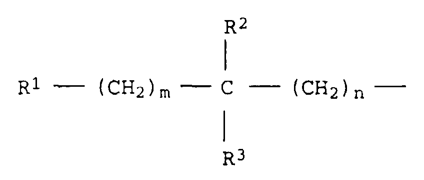
B für 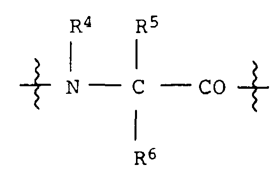
C für 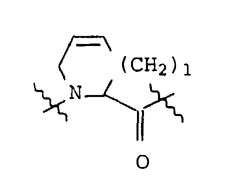
D für 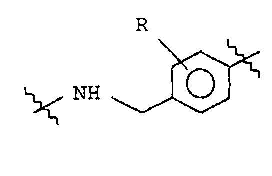 steht
und E die in den Schemata angegebene Bedeutung besitzt. Die Reste R, R¹, R², R³, R⁴, R⁵ und R⁶ sowie l, m und n haben die oben angegebene Bedeutung.

Die Bausteine A, B, C und D werden vorzugsweise vorher separat aufgebaut und in geeignet geschützter Form (siehe Schema I-III) eingesetzt.

Die Verbindungen der Formel I lassen sich ausgehend von den entsprechend geschützten Bausteinen A, B, C, D und E nach Schema I-III herstellen. 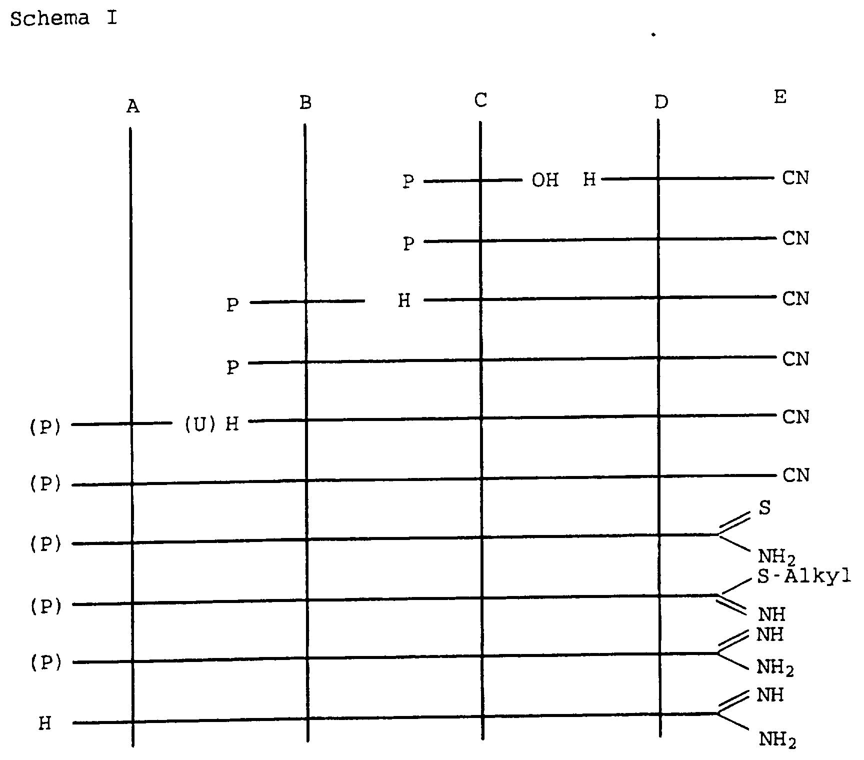 (P = Schutzgruppe, (P) = Schutzgruppe oder H, (U) = Abgangsgruppe oder gegebenenfalls Aldehyd bzw. Keton, siehe nachfolgenden Text)

Schema I beschreibt den linearen Aufbau des Moleküls I durch Kupplung des Amins H-D-CN mit der N-geschützten Aminosäure P-C-OH zu P-C-D-CN, Abspaltung der N-terminalen Schutzgruppe zu H-C-D-CN, Kupplung mit der N-geschützten Aminosäure P-B-OH zu P-B-C-D-CN, Abspaltung der Schutzgruppe P zu H-B-C-D-CN, anschließende Alkylierung mit dem gegebenenfalls geschützten (P)-A-U-Baustein (U = Abgangsgruppe) oder reduktive Aminierung mit (P)-A'-U (U = Aldehyd, Keton) oder Michael-Addition mit einem geeignetem (P)-A"-C=C-Derivat zu (P)-A-B-C-D-CN. Die Umwandlung der Nitrilfunktion in die Amidingruppe erfolgt entweder über die klassische Pinner-Synthese (R. Boder, D.G. Neilson, Chem. Rev. 1962, 61, 179) oder über eine modifizierte Pinner-Synthese, die über Iminothioestersalze als Zwischenstufe abläuft (H. Vieweg et al., Pharmazie 1984, 39, 226) oder direkt nach der Methode von A. Eschenmoser Helv. Chimica Acta 69 (1986) 1224. Anschließend werden im Molekül noch vorhandene Schutzgruppen vorzugsweise durch saure Hydrolyse abgespalten.

Wird der Baustein D als H-D-CONH₂ in der Synthese eingebaut, so erfolgt auf einer der geschützten Zwischenstufen die Dehydratisierung der Amid- zur Nitrilfunktion.

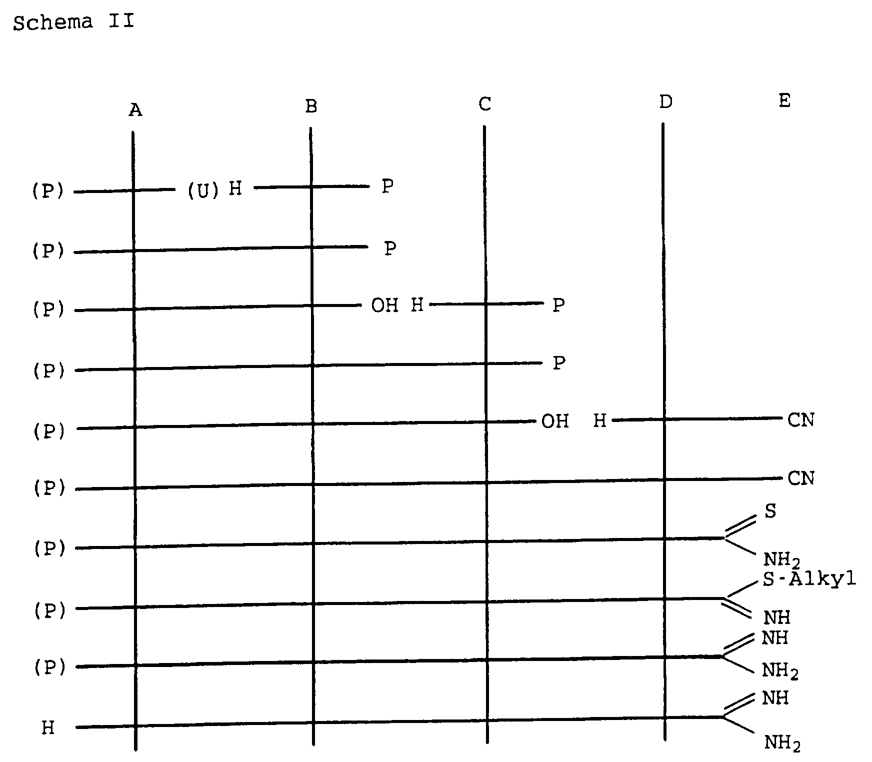

Schema II beschreibt den linearen Aufbau des Moleküls I durch Alkylierung, reduktive Aminierung oder Michael-Addition von H-B-P an entsprechend geeignete gegebenenfalls geschützte A-Bausteine zu (P)-A-B-P, Abspaltung der C-terminalen Schutzgruppe zu (P)-A-B-OH, Kupplung mit H-C-P zu (P)-A-B-C-P, Abspaltung der C-terminalen Schutzgruppe zu (P)-A-B-C-OH, Kupplung mit H-D-CN zu (P)-A-B-C-D-CN und Umsetzung dieses Zwischenprodukts zum Endprodukt analog Schema I.

Bei Verbindungen (P)-A-B-P mit noch freier NH-Funktion an B muß diese vor Abspaltung der C-terminalen Schutzgruppe noch mit einer geeigneten Schutzgruppe versehen werden. Die jeweils verwendeten Schutzgruppen müssen orthogonal zueinander sein.

Alternativ zum H-D-CN-Baustein kann auch H-D-CONH₂, H-D-C(NH)NH₂, H-D-C(NP)NH₂, H-D-C(NP)NHP eingesetzt werden, wobei im ersten Fall das gekuppelte Zwischenprodukt (P)-A-B-C-D-CONH₂ zu (P)-A-B-C-D-CN dehydratisiert wird.

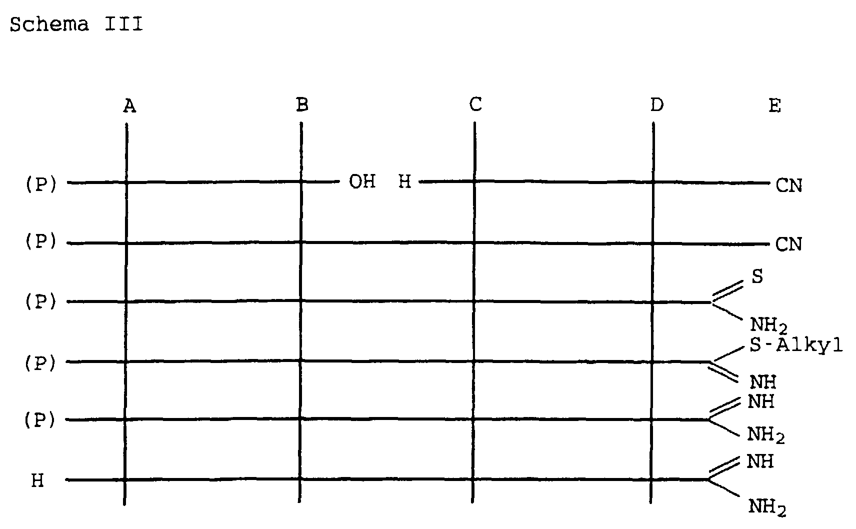

Schema III beschreibt einen sehr effizienten Weg zur Darstellung der Verbindungen I durch eine konvergente Synthese. Die entsprechend geschützten Bausteine (P)-A-B-OH und H-C-D-CN werden miteinander gekuppelt und das entstandene Zwischenprodukt (P)-A-B-C-D-CN analog Schema I zum Endprodukt umgesetzt.

Als N-terminale Schutzgruppen werden Boc, Cbz oder Fmoc, vorzugsweise Boc eingesetzt, C-terminale Schutzgruppen sind Methyl, tert.-Butyl und Benzyl. Sind mehrere Schutzgruppen im Molekül vorhanden, so müssen diese orthogonal zueinander sein, wenn sie nicht gleichzeitig abgespalten werden sollen. Enthalten die Zwischenprodukte den Baustein C, sind Cbz- und Benzylschutzgruppen ungeeignet.

Die erforderlichen Kupplungsreaktionen sowie die üblichen Reaktionen der Schutzgruppeneinführung und -abspaltung werden nach Standardbedingungen der Peptidchemie durchgeführt (siehe M. Bodanszky, A. Bodanszky "The Practice of Peptide Synthesis", 2. Auflage, Springer Verlag Heidelberg, 1994).

Boc-Schutzgruppen werden mittels Dioxan/HCl oder TFA/DCM, Cbz-Schutzgruppen hydrogenolytisch oder mit HF abgespalten. Die Verseifung von Esterfunktionen erfolgt mit LiOH in einem alkoholischen Lösungsmittel oder in Dioxan/Wasser. t-Butylester werden mit TFA gespalten.

Die Reaktionen wurden mittels DC kontrolliert, wobei üblicherweise folgende Laufmittel benutzt wurden:

| | | |
|---|---|---|
| A. | DCM/MeOH | 95:5 |
| B. | DCM/MeOH | 9:1 |
| C. | DCM/MeOH | 8:2 |
| D. | DCM/MeOH/50 %ig HOAc | 40:10:5 |
| E. | DCM/MeOH/50 %ig HOAc | 35:15:5 |

Sofern säulenchromatographische Trennungen erwähnt werden, waren dies Trennungen über Kieselgel, für die die oben genannten Laufmittel verwendet wurden.

Reversed phase HPLC Trennungen wurden mit Acetonitril/Wasser und HOAc Puffer durchgeführt.

Die Ausgangsverbindungen lassen sich nach folgenden Methoden herstellen:

Als Bausteine A werden für die Alkylierung z.B. α-Bromessigsäure-tert.-butylester, β-Brompropionsäure-tert.-butylester, α-Brompropionsäure-tert.-butylester, γ-Brombuttersäure-tert.-butylester, α-Brombuttersäure-tert.-butylester, THP-geschütztes Bromethanol, THP-geschütztes γ-Brompropanol, α-Brom-γ-butyrolacton, für die reduktive Aminierung z.B. Dihydroxyaceton, Acetondicarbonsäure-di-tert.-butylester und für die Michael-Addition z.B. Acrylsäure-tert.-butylester, Methacrylsäure-tert.-butylester, Fumarsäure-tert.-butylester eingesetzt. Die genannten tert.-Butylester werden, soweit sie nicht käuflich zu erwerben sind, analog G. Uray, W. Lindner Tetrahedron 1988, 44, 4357-62 aus den entsprechenden Carbonsäuren hergestellt.

### B-Bausteine:

Für die allgemeine und spezielle Synthese von Aminosäuren stehen in der Literatur vielfältige Möglichkeiten zur Verfügung. Eine Übersicht hierzu bietet u.a. Band E16d/Teil 1 - Houben-Weyl, S. 406 ff.

Häufig eingesetzte Edukte waren Benzophenoniminessigsäureethylester, Acetamidomalonsäurediethylester und Isonitrilessigsäureethylester.

Die Darstellung verschiedener Glycin-und Alaninderivate erfolgte z.B. ausgehend von Isonitrilessigsäureethylester und einem entsprechenden Keton bzw. Aldehyd (siehe H.-J. Prätorius, J. Flossdorf, M.-R. Kula Chem. Ber. 1975, 108, 3079).

Die Synthesen von 2-Norbonylglycin, Adamantylalanin, γ-Methylcyclohexylalanin, 4-Isopropylcyclohex-1-yl-alanin, 4-Methylcyclohex-1-yl-alanin und 4-Methylcyclohex-1-ylglycin wurden über die entsprechenden 2-Formylamino-acrylsäureethylester (U. Schöllkopf und R. Meyer, Liebigs Ann. Chem. 1977, 1174) ausgehend von Isocyanessigsäureethylester mit den jeweiligen Carbonylverbindungen 2-Norbornanon, 1-Formyladamantan, 1-Formyl-1-methyl-cyclohexan, 1-Formyl-4-isopropyl-cyclohexan, 1-Formyl-4-methyl-cyclohexan und 4-Methylcyclohexanon nach folgenden allgemeinen Vorschriften durchgeführt:

### Allgemeine Arbeitsvorschrift zur Synthese der 2-Formylaminoacrylsäureethylester

Zu 100 mMol Kalium-tert.-butylat in 150 ml THF tropft man bei 0 bis -10°C die Lösung von 100 mMol Isocyanessigsäureethylester in 50 ml THF. Nach 15 min fügt man bei gleicher Temperatur 100 mMol der entsprechenden Carbonylverbindung in 50 ml THF zu, läßt die Reaktionsmischung langsam auf RT ansteigen und zieht das Lösungsmittel am Rotationsverdampfer ab. Der Rückstand wird mit 50 ml Wasser, 100 ml Essigsäure und 100 ml DCM vermischt und das Produkt mit DCM extrahiert. Die DCM-Phase wird über Na₂SO₄ getrocknet und das Lösungsmittel am Rotationsverdampfer abgezogen. Die fast rein anfallenden Produkte können im Bedarfsfall säulenchromatographisch über Kieselgel (Laufmittel: Gemische aus Ether/Petrolether) weiter gereinigt werden.

### Allgemeine Vorschrift der Aminosäurehydrochloride ausgehend von den 2-Formylamino-acrylsäureethylestern

100 mMol der 2-Formylamino-acrylsäureethylester werden mit Pd/C (10 %)-Wasserstoff in 200 ml Eisessig bis zur vollständigen Umsetzung hydriert. Dann wird der Katalysator abfiltriert, die Essigsäure so weit wie möglich am Rotationsverdampfer abgezogen und der Rückstand in 200 ml halbkonzentrierter Salzsäure 5 h zum Rückfluß erhitzt. Man zieht die Salzsäure am Rotationsverdampfer ab, trocknet das Produkt bei 50°C im Vakuum und wäscht mehrmals mit Ether nach. Die Hydrochloride fallen als schwach gefärbte Kristalle an.

Ausgehend von 16,5 g (150 mMol) 2-Norbornanon erhielt man 26,6 g 2-Norbonylglycin-hydrochlorid. Ausgehend von 19,7 g (120 mMol) 1-Formyladamantan erhielt man 26,0 g Adamantylalanin-hydrochlorid. Ausgehend von 12,6 g (100 mMol) 1-Formyl-1-methyl-cyclohexan erhielt man 16,6 g γ-Methylcyclohexylalanin-hydrochlorid. Ausgehend von 16,8 g (150 mMol) 4-Methylcyclohexanon erhielt man 25,9 g (4-Methyl)-cyclohexylglycin-hydrochlorid.

Ausgehend von 15 g trans-1-Formyl-4-methylcyclohexan erhielt man 18 g trans-4-Methylcyclohex-1-yl-alanin-hydrochlorid.

Ausgehend von 9 g 3,3-Dimethyl-1-formylcyclohexan erhielt man 10 g 3,3-Dimethylcyclohex-1-yl-alaninhydrochlorid.

Der für die Synthese benötigte Aldehyd, 1-Formyl-3,3-dimethylcyclohexan, wurde in Anlehnung an Moskal und Leusen (Rec. Trav. Chim. Pays-Bas 1987, 106, 137-141) dargestellt:

Eine Lösung von n-Butyl-lithium in n-Hexan (72 ml, 115 mmol) wurde innerhalb von 10 min bei -60°C zu einer gerührten Lösung von Diethylisocyanomethylphosphonat (17 ml, 105 mmol) in 280 ml wasserfreiem Diethylether getropft. Die entstandene Suspension wurde 15 min bei -60°C nachgerührt und innerhalb von 10 min mit einer Lösung von 3,3-Dimethylcyclohexanon (13 g, 105 mmol) in 100 ml wasserfreiem Diethylether versetzt, wobei die Temperatur unter -45°C gehalten wurde. Man ließ das Reaktionsgemisch auf 0°C kommen, rührte 90 min bei dieser Temperatur und gab vorsichtig 150-200 ml 38 %ige wäßrige Salzsäure hinzu. Zur vollständigen Hydrolyse wurde 15 h lang bei Raumtemperatur heftig gerührt. Man trennte die organische Phase ab, wusch sie mit je 200 ml Wasser, gesättigter Natriumhydrogencarbonat-Lösung und gesättigter Natriumchlorid-Lösung. Man trocknete über Magnesiumsulfat, filtrierte ab und engte am Rotationsverdampfer ein, um die Lösungsmittel zu entfernen. Der erhaltene Rückstand wurde ohne weitere Reinigung als Ausgangsmaterial für die Synthese der Aminosäure eingesetzt.

### Boc-(D)-α-methyl-cyclohexylalanin:

3,4 g (12,2 mMol) Boc-(D)-α-Methyl-Phe-OH wurden in 100 ml MeOH bei 50°C in Gegenwart von 250 mg 5-%igem Rh auf Al₂O₃ 24 h bei 10 bar mit Wasserstoff hydriert. Man erhielt nach Filtration und Abziehen des Lösungsmittels 2,8 g Boc-(D)-α-Methyl-Cha-OH.

¹H-NMR (DMSO-d⁶, δ in ppm): 12 (sehr breites Signal, COOH); 1,7-0,8 (25 H; 1,35 (s, Boc), 1,30 (s,Me))

Boc-(3-Ph)-Pro-OH wurde analog einer Vorschrift von J.Y.L. Chung et al. (J.Y.L. Chung et al. J.Org.Chem. 1990, 55, 270) synthetisiert.

### Darstellung von Boc-(D,L)-Dch-OH:

Boc-(D,L)-Dpa-OH (lmmol) wurde in 12 ml MeOH zusammen mit katalytischen Mengen von 5 % Rh/Al₂O₃ bei 5 bar hydriert. Nach Filtration und Entfernen des Solvens im Vakuum erhielt man das Produkt in quantitativer Ausbeute.

### Darstellung von H-(D,L)-Chea-OH:

4,0 g Cycloheptylmethylmethansulfonat (19,39 mMol), hergestellt aus Cycloheptylmethanol und Methansulfonsäurechlorid, wurden zusammen mit 4,9 g Benzophenoniminglycinethylester (18,47 mMol), 8,9 g trockenem fein gepulvertem Kaliumcarbonat (64,65 mMol) und 1 g Tetrabutylammoniumbromid (3 mMol) in 50 ml trockenem Acetonitril 10 h in Inertgasatmosphäre auf Rückfluß erhitzt. Danach wurde das Kaliumcarbonat abfiltriert, das Filtrat zur Trockene eingedampft, und das Rohprodukt direkt mit 20 ml 2N Salzsäure in 40 ml Ethanol 1,5 h unter Rühren bei RT hydrolisiert. Nach Verdünnen der Reaktionslösung wurde mit Essigester im sauren Bereich Benzophenon extrahiert, anschließend im alkalischen Bereich (pH = 9) H-(D,L)-Chea-OEt mit DCM extrahiert, die Lösung über Magnesiumsulfat getrocknet und einrotiert. Ausbeute 3,7 g 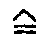 95 % der Theorie.

Die Darstellung von (D)-(1,4-Cyclohexadien-1-yl)ala-OH erfolgte nach G. Zivilichovsky, V. Gurvich J. Chem. Soc. Perkin Trans I 19 (1995) 2509-15.

Die Darstellung von H-(D,L)-β,β-Me₂Cha-OH erfolgte nach U. Schöllkopf, R. Meyer, L. Ann. Chem. 1977, 1174-82.

Die genannten Aminosäuren wurden nach allgemein bekannten Verfahren mit Di-tert.-butyl-dicarbonat in Wasser/Dioxan in die jeweils Boc-geschützte Form überführt und anschließend aus Essigester/Hexan-Gemischen umkristallisiert oder säulenchromatographisch über Kieselgel (Laufmittel: Essigester/Petrolether-Gemische) gereinigt.

Die Boc-geschützten Aminosäuren wurden als B-Bausteine entsprechend Schema I eingesetzt.

Die genannten Aminosäuren wurden als B-Bausteine teilweise auch in die entsprechenden Benzylester überführt, mit den entsprechend geschützten A-Bausteinen verknüpft und die Verbindungen mit noch freier N-H-Funktion anschließend mit einer Boc-Gruppe geschützt. Die Benzylestergruppe wurde abhydriert und der Baustein A-B-OH durch Kristallisation, Salzfällung bzw. Säulenchromatographie gereinigt. Dieser Weg ist exemplarisch für tBuOOC-CH₂-(Boc)(D)Cha nachfolgend beschrieben.

### Synthese von (D)-Cyclohexylalaninbenzylester:

Eine Suspension von 100 g (481 mmol) D-Cyclohexylalanin-hydrochlorid, 104 g (962 mmol) Benzylalkohol und 110 g (577 mmol) p-Toluolsulfonsäuremonohydrat in 2200 ml Toluol wurde am Wasserabscheider langsam zum Rückfluß erhitzt. In einem Temperaturbereich von 80-90°C beobachtete man Chlorwasserstoffentwicklung sowie das Auflösen der Suspension zu einer klaren Lösung. Als sich kein Wasser mehr abschied (ca. 4 h), destillierte man 500 ml Toluol ab, ließ die Reaktionsmischung über Nacht abkühlen, filtrierte den entstandenen Rückstand ab und wusch zweimal mit je 1000 ml Hexan nach. Der erhaltene Rückstand (195 g) wurde sodann in 2000 ml Dichlormethan aufgeschlämmt, mit 1000 ml Wasser versetzt und unter Rühren durch sukzessive Zugabe von 50 %iger Natronlauge auf pH 9-9,5 eingestellt. Man trennte die organische Phase ab, wusch sie zweimal mit je 500 ml Wasser, trocknete sie über Natriumsulfat, filtrierte vom Trockenmittel ab und engte das Filtrat ein, wodurch man 115 g (94 %) des Produktes als helles Öl erhielt.

### N-(tert.-butyloxycarbonylmethyl)-(D)-cyclohexylalaninbenzylester:

115 g (440 mmol) (D)-Cyclohexylalaninbenzylester wurden in 2000 ml Acetonitril gelöst, bei Raumtemperatur mit 608 g (4,40 mol) Kaliumcarbonat und 94 g (484 mmol) Bromessigsäure-tert.-butylester versetzt und 3 Tage bei dieser Temperatur gerührt. Man filtrierte vom Carbonat ab, wusch mit Acetonitril nach, engte die Mutterlauge ein (30°C, 20 mbar), nahm den Rückstand in 1000 ml Methyl-tert.-butylether auf und extrudierte die organische Phase mit 5 %iger Zitronensäure und gesättigter Natriumhydrogencarbonat-Lösung. Man trocknete die organische Phase über Natriumsulfat, filtrierte vom Trockenmittel ab, engte ein und setzte das erhaltene Öl (168 g) direkt in die folgende Reaktion ein.

### N-Boc-N- (tert.-butyloxycarbonylmethyl)-(D)-cyclohexylalaninbenzylester:

Das in vorheriger Synthese erhaltene Öl (168 g, 447 mmol) wurde in 1400 ml Acetonitril gelöst, mit 618 g (4,47 mol) Kaliumcarbonat-Pulver und 107 g (492 mmol) Di-tert.-butyldicarbonat versetzt und 6 Tage bei Raumtemperatur gerührt. Man saugte das Kaliumcarbonat ab, wusch mit ca. 1000 ml Acetonitril nach und engte das Filtrat ein. Man erhielt 230 g des gewünschten Produkts.

### N-Boc-N-(tert.-butyloxycarbonylmethyl)-(D)-cyclohexylalanincyclohexylammoniumsalz:

115 g N-Boc-N-(tert.-butyloxycarbonylmethyl)-(D)-cyclohexylalaninbenzylester wurden in 1000 ml reinem Ethanol gelöst und bei 25-30°C in Gegenwart von 9 g 10 %igem Pd auf Aktivkohle 2 h bei Normaldruck mit Wasserstoff hydriert. Nach Filtration und Entfernen des Lösungsmittels am Rotationsverdampfer erhielt man 100 g (260 mmol) eines gelben Öls, das man in 1600 ml Aceton aufnahm und zum Rückfluß erhitzte. Man entfernte das Heizbad und gab über einen Tropftrichter zügig eine Lösung von 27 g (273 mmol) Cyclohexylamin in Aceton hinzu. Beim Abkühlen der Reaktionsmischung auf Raumtemperatur kristallisierte das gewünschte Salz aus. Man filtrierte den Feststoff ab, wusch mit 200 ml Aceton nach und kristallisierte zur endgültigen Reinigung noch einmal aus Aceton um. Nach Trocknung des Rückstandes im Vakuumtrockenschrank bei 30°C erhielt man 70,2 g des gewünschten Salzes als weißes Pulver.

Das als C-Baustein eingesetzte (L)-3,4-Dehydroprolin ist käuflich zu erwerben, die (D,L)-4,5-Dehydropipecolinsäure läßt sich J. Org. Chem. 25 (1960), 489 oder C. Herdeis, W. Engel Arch. Pharm. 326 (1993), 297 herstellen und anschließend mit Boc₂O in Boc(D,L)-Dep-OH überführen.

Die Synthese der D-Bausteine ist in DE 444 33 90 beschrieben.

### Beispiel 1

### N-Hydroxycarbonyl-methylen-(D)-cyclohexylalanyl-3,4-dehydroprolyl-(4-amidino)-benzylamid:

### Boc-3,4-Dehydroprolyl-4-cyanobenzylamid:

Boc-3,4-Dehydroprolin (4,7 g, 22,0 mmol) und 4-Cyanobenzylamin (4,1 g, 24,2 mmol; DE 444 33 90) wurden in Dichlormethan (25 ml) gelöst. Die Lösung wurde auf 0°C gekühlt und mit Ethyldiisopropylamin (26,4 ml, 154 mmol) versetzt. Anschließend wurde 50 %iges Propanphosphonsäureanhydrid in Essigsäureethylester (23,3 ml, 110 mmol) langsam zugetropft. Es wurde 1 h bei 0°C und 30 min bei Raumtemperatur nachgerührt. Die Reaktionsmischung wurde mit Dichlormethan verdünnt und mit verdünnter Natriumhydrogensulfatlösung (3 x), verdünnter Natriumhydrogencarbonatlösung (3 x), gesättigter Natriumchloridlösung gewaschen. Nach Trocknen über Natriumsulfat wurde im Wasserstrahlvakuum eingeengt. Man erhielt 7,47 g Rohprodukt.

### 3,4-Dehydroprolyl-4-cyanobenzylamid:

Das aus dem vorangegangenen Versuch erhaltene Rohprodukt von Boc-3,4-Dehydroprolyl-4-cyanobenzylamid (7,47 g) wurde in Dichlormethan (88 ml) gelöst und mit etherischer Salzsäurelösung (88 ml, 5 M) versetzt. Anschließend wurde 1,5 h bei Raumtemperatur gerührt. Das Lösungsmittel wurde im Wasserstrahlvakuum abdestilliert. Der Rückstand wurde 2 x mit Dichlormethan versetzt und das Lösungsmittel im Wasserstrahlvakuum erneut abdestilliert. Anschließend wurde 2 x mit Diethylether ausgerührt. Man erhielt 5,32 g Rohprodukt.

### N-(tert.-Butoxycarbonyl-methylen)-(N-BOC)-(D)-cyclohexylalanyl-3,4-dehydroprolyl-4-cyanobenzylamid:

tBuOOC-CH₂-(Boc)-(D)-Cha-OH (7,59 g, 19,68 mmol) und H-Pyr-4-cyanobenzylamid (5,19 g, 19,68 mmol) wurden in Dichlormethan (100 ml) gelöst und mit Ethyldiisopropylamin (12,72 g, 98,4 mmol) versetzt. Die Lösung wurde auf 0°C gekühlt und 50 %ige Propanphosphonsäureanhydrid in Essigsäureethylester (20 ml) in 20 min zugetropft nach 3 h Rühren bei 0-10°C wurde mit Dichlormethan (100 ml) verdünnt, mit 10 %iger Natriumhydrogensulfatlösung (3 x), gesättigter Natriumhydrogencarbonatlösung (2 x) und Wasser gewaschen. Nach Trocknen über Natriumsulfat wurde das Lösungsmittel im Wasserstrahlvakuum abdestilliert. Man erhielt 13,28 g als leicht bräunliches Öl.

### N-(tert.-Butoxycarbonyl-methylen)-(N-Boc)-(D)-cyclohexylalanyl-3,4-dehydroprolyl-4-aminothiocarbonylbenzylamid:

Das aus dem vorangegangenen Versuch erhaltene Rohprodukt von t-BuOOC-CH₂-(Boc)-(D)-Cha-Pyr-4-cyano-benzylamid (13,28 g) wurde in Pyridin (70 ml) und Triethylamin (12 ml) gelöst, auf 0°C gekühlt und die Lösung mit Schwefelwasserstoff gesättigt (Lösung färbte sich grün). Anschließend wurde 48 h bei Raumtemperatur gerührt. Der überschüssige Schwefelwasserstoff wurde mit Stickstoff verdrängt und das Löungsmittel im Wasserstrahlvakuum abdestilliert. Der Rückstand wurde in Diethylether gelöst und 3 x mit 20 %iger Natriumhydrogensulfatlösung, gesättigter Natriumhydrogencarbonatlösung (2 x) und Wasser gewaschen. Nach Trocknen über Natriumsulfat wurde das Lösungsmittel im Wasserstrahlvakuum abdestilliert. Das Rohprodukt (14,3 g) wurde mittels Flashchromatographie (Kieselgel, Gradient von Dichlormethan bis Dichlormethan:Methanol = 50:1) gereinigt. Ausbeute: 13,3 g (leicht lösungsmittelhaltig).

### N-(tert.-Butoxycarbonyl-methylen)-(N-Boc)-(D)-cyclohexylalanyl-3,4-dehydroprolyl-4-S-methylimiinocarbonylbenzylamid:

Das aus dem vorangegangenen Versuch erhaltene t-BuOOC-CH₂-(Boc)-(D)-Cha-Pyr-aminothiocarbonyl-benzylamid (13,3 g) wurde in Dichlormethan (135 ml) mit Methyliodid (7,97 ml, 126,90 mmol) versetzt. Nach 24 h Rühren bei Raumtemperatur wurde das Lösungsmittel im Wasserstrahlvakuum abdestilliert. Man erhielt 15,73 g eines leicht gelblichen Öls.

### N- (tert.-Butoxycarbonyl-methylen-(N-Boc)-(D)-cyclohexylalanyl-3,4-dehydroprolyl-4-amidinobenzylamid:

Das aus dem vorangegangenen Versuch erhaltene Rohprodukt von t-BuOOC-CH₂-(Boc)-(D)-Cha-Pyr-4-S-methyliminocarbonyl-benzylamidhydroiodid (15,73 g) wurde in Acetonitril (1290 ml) gelöst und mit Ammoniumacetat (3,25 g, 42,3 mmol) versetzt. Anschließend wurde 1,5 h auf 50°C erhitzt. Die Reaktionsmischung wurde im Wasserstrahlvakuum eingeengt und mit Dichlormethan versetzt. Die ausgefallenen Salze wurden abfiltriert und das Filtrat im Wasserstrahlvakuum eingeengt. Man erhielt 15,17 g eines gelblichen Schaumes. Das Rohprodukt wurde über einen Ionenaustauscher (Fluka, Bestell-Nr. 00402, Acetat auf polymerem Träger) in das Acetatsalz überführt. Ausbeute: 13,3 g.

### N-(Hydroxycarbonyl-methylen)-(D)-cyclohexylalanyl-3,4-dehydroprolyl-(4-amidino)-benzylamid:

t-BuOOC-CH₂-(Boc) - (D) -Cha-Pyr-4-amidino-benzylamid-hydroacetat (13,3 g) wurde in Dichlormethan (200 ml) gelöst und mit etherischer HCl (45 ml) versetzt. Nach 2 h Rühren bei Raumtemperatur wurde im Wasserstrahlvakuum bis zur Trockene eingedampft. Der Rückstand wurde 2 x mit Dichlormethan versetzt und das Lösungsmittel im Wasserstrahlvakuum abdestilliert. Man erhielt 11,6 g Rohprodukt.

Ein Teil des Rohprodukts (3 g) wurde über einen Ionenaustauscher (Fluka, Bestell-Nr. 00402, Acetat auf polymerem Träger) in das Acetatsalz überführt. Das so erhaltene Produkt (2,9 g) wurde mittels Flashchromatographie gereinigt (Kieselgel, Gradient von Dichlormethan:Methanol = 4:1 über Dichlormethan:Methanol:50 %ige Essigsäure = 40:10:2 bis Dichlormethan:Methanol:50 %ige Essigsäure = 35:15:5). Man erhielt ein gelbliches Öl, das in Wasser gelöst wurde. Nach Filtration wurde das Filtrat gefriergetrocknet. Ausbeute: 2,13 g eines farblosen Feststoffes. FAB-MS (M+H⁺): 456.

Analog Beispiel 1 wurden hergestellt:
2. N-(Hydroxycarbonyl-methylen)-(D)-cyclohexylglycyl-3,4-dehydroprolyl-(4-amidino)-benzylamid:
   FAB-MS (M+H⁺): 442
3. N-(Hydroxycarbonyl-methylen)-(D,L)-cycloheptylalanyl-3,4-dehydroprolyl-(4-amidino)-benzylamid:
   FAB-MS (M+H⁺): 470
4. N-(Hydroxycarbonyl-methylen)-(D)-tert.-butylalanyl-3,4-dehydroprolyl-(4-amidino)-benzylamid:
   FAB-MS (M+H⁺): 430
5. N-(Hydroxycarbonyl-methylen)-(D)-cyclohexylalanyl-4,5-dehydro-pipecolyl-(4-amidino)-benzylamid:
   FAB-MS (M+H⁺): 470

Die antithrombotische Wirkung der neuen Verbindungen wurde am arteriovenösen Shunt an der Ratte gezeigt. In diesem Experiment dient eine Glaskapillare in einem arteriovenösen Shunt als künstliche thrombogene Oberfläche und löst eine Thrombose aus. Die narkotisierte (Urethan 25 %, 2 x 8 mg/kg i.p.) Ratte wird in Rückenlage auf einer temperierten (37°C) Wärmebank fixiert. In die freipräparierte rechte A. carotis und V. jugularis werden kurze Polyethylene-Katheter (Portex, PE 50) implantiert, mit physiol. NaCl-Lösung gefüllt und durch Klemmen verschlossen. Die freien Enden der Katheter werden durch eine 20,0 mm lange Glaskapillare (Innendurchmesser 1,0 mm) verbunden, die als thrombogene Oberfläche wirkt. Die Applikation der Prüfsubstanz kann i.v., s.c., p.o. oder als Infusion erfolgen. Nach der gewünschten Inkubationszeit mit der Prüfsubstanz oder Lösungsmittel (Kontrolle) wird der Shunt durch Entfernen der Klemmen geöffnet. Der Blutstrom durch den Shunt führt zu einem schnellen Anstieg der Shunttemperatur, die an der Mitte der Glaskapillare gemessen wird. Die Erhöhung von Raumtemperatur auf Körpertemperatur ist ein Indikator für die Durchgängigkeit des Shunts. Die Temperatur wird bis zum Verschluß des Shunts kontinuierlich aufgezeichnet. Bei Öffnung des Shunts und am Ende des Experiments werden zusätzlich Blutproben zur Bestimmung der anti-FIIa-Aktivität im Plasma entnommen.

### Pharmakokinetik und Gerinnungsparameter in Hunden

Die Testsubstanzen werden unmittelbar vor der Verabreichung an wache Mischlingshunde in isotonischer Salzlösung gelöst. Die Applikationsvolumina betragen 0,1 ml/kg für die intravenöse Bolus-Injektion und 1 ml/kg für die orale Verabreichung, die per Schlundsonde erfolgt. Vor sowie 5, 10, 20, 30, 45, 60, 90, 120, 180, 240, 300 und 360 min (bei Bedarf nach 420, 480 min und 24 h) nach intravenöser Applikation von 1,0 mg/kg bzw. vor sowie 10, 20, 30, 60, 120, 180, 240, 300, 360, 480 min und 24 h nach oraler Gabe von 4,64 mg/kg werden Proben venösen Blutes (2 ml) in Citrat-Röhrchen genommen. Direkt nach der Probennahme wird die Ecarinzeit (ECT = ecarin clotting time) im Ganzblut bestimmt. Nach der Präparation des Plasmas durch Zentrifugation werden die Plasma-Thrombinzeit und die aktivierte partielle Thromboplastinzeit (APTT = aktivated partial thromboplastin time) mit Hilfe eines Koagulometers bestimmt.

Zusätzlich werden die anti-F IIa-Aktivität (ATU/ml) und die Konzentration der Substanz durch ihre anti-F IIa-Aktivität im Plasma mittels chromogenem (S-2238) Thrombin-Assay bestimmt, wobei Eichkurven mit r-Hirudin und der Testsubstanz eingesetzt wurden.

Die Plasmakonzentration der Testsubstanz ist Grundlage zur Berechnung der pharmakokinetischen Parameter: Zeit der maximalen Plasmakonzentration (T max), maximale Plasmakonzentration; Plasma-Halbwertzeit, T_{0,5}; Fläche unter der Kurve (AUC); resorbierter Teil der Testsubstanz (F).

### Gerinnungs-Parameter:

Ecarinzeit (ECT = ecarin clotting time): 100 µl citratbehandeltes Blut werden 2 min bei 37°C in einem Koagulometer inkubiert (CL 8, Kugel-Typ, Bender & Hobein, München, BRD). Nach Zugabe von 100 µl vorgewärmtem (37°C) Ecarin-Reagenz (Pentapharm) wird die Zeit bis zur Bildung eines Fibrin-Clots bestimmt.

Aktivierte Thromboplastinzeit (APTT = activated thromboplastin time): 50 µl citratbehandeltes Plasma und 50 µl des PTT-Reagenzes (Pathrombin, Behring) werden gemischt und 2 min bei 37°C in einem Koagulometer inkubiert (CL 8, Kugel-Typ, Bender & Hobein, München, BRD). Nach der Zugabe von 50 µl vorgewärmtem (37°C) Calciumchlorid wird die Zeit bis zur Bildung eines Fibrin-Clots bestimmt.

Thrombinzeit (TT): 100 µl citratbehandeltes Plasma wird 2 min bei 37°C in einem Koagulometer inkubiert (CL 8, Kugel-Typ, Bender & Hobein, München, BRD). Nach der Zugabe von 100 µl vorgewärmtem (37°C) Thrombin-Reagenz (Boehringer Mannheim) wurde die Zeit bis zur Bildung eines Fibrin-Clots bestimmt.

In diesen Versuchen zeigten die neuen Verbindungen eine gute Wirkung.

## Patentansprüche

1. Verbindungen der Formel I 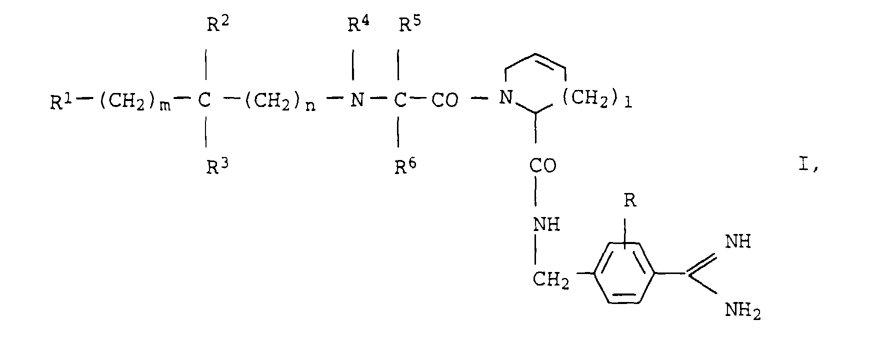 worin die Reste R, R¹, R², R³, R⁴, R⁵ und R⁶ sowie l, m und n folgende Bedeutungen besitzen:
l 0 oder 1,
m 0,1 oder 2,
n 0,1 oder 2,
R H oder C₁₋₄-Alkyl-,
R¹ HOOC-, C₁₋₆-Alkyl-OOC-, Benzyl-OOC- oder -OH,
R² H-, C₁₋₄-Alkyl- oder R¹-(CH₂)ₘ-,
R³ H- oder C₁₋₄-Alkyl-, welches durch -OH oder -COOH substituiert sein kann,
R⁴ H-, C₁₋₄-Alkyl-, HOOC-C₁₋₄-alkylen-,
R⁵ C₁₋₈-Alkyl-, Cycloalkyl-(CR⁸R⁹)ᵣ-, (r = 0 oder 1, R⁸, R⁹ = H-, Cycloalkyl- oder C₁₋₄-Alkyl-), worin bis zu vier CH₂-Gruppen des Cycloalkylrestes unabhängig voneinander durch CR¹⁰R¹¹ (R¹⁰ = H-oder C₁₋₄-Alkyl-, R¹¹ = C₁₋₄-Alkyl-) und/oder die an CR⁸R⁹ gebundene CH-Gruppe des Cycloalkylrestes durch CR¹² (R¹² = C₁₋₄-Alkyl-) ersetzt sein können und/oder eine oder zwei C-C-Einfachbindung(en) im Ring durch eine C=C-Doppelbindung ersetzt sein können,
R⁶ H-, C₁₋₄-Alkyl- oder
R⁴ und R⁵ zusammen -CH₂-CH₂-CH(R⁷)-, (R⁷ = H-, Phenyl- oder Cyclohexyl-)
R² und R⁵ zusammen -CH₂-CH₂- oder -CH₂-CH₂-CH₂-, worin ein Wasserstoffatom durch C₁₋₄-Alkyl-, Phenyl- oder Cycloalkyl- ersetzt sein kann,
sowie deren Salze mit physiologisch verträglichen Säuren.

2. Verbindungen der Formel I gemäß Anspruch 1 zur Verwendung bei der Bekämpfung von Krankheiten.

3. Verwendung der Verbindungen der Formel I gemäß Anspruch l zur Herstellung von Arzneimitteln gegen:
• Krankheiten, deren Pathomechanismus direkt oder indirekt auf der proteolytischen Wirkung von Thrombin beruht,
• Krankheiten, deren Pathomechanismus auf der thrombinabhängigen Aktivierung von Rezeptoren und Signaltransduktionen beruht,
• Krankheiten, die mit Stimulation oder Inhibition von Genexpressionen in Körperzellen einhergehen,
• Krankheiten, die auf der mitogenen Wirkung von Thrombin beruhen,
• Krankheiten, die auf einer thrombinabhängigen Kontraktilitäts- und Permeabilitätsveränderung von Epithelzellen beruhen,
• thrombinabhängige, thromboembolische Ereignisse,
• disseminierte intravasale Koagulation,
• Reokklusion und zur Verkürzung der Reperfusionszeit bei Komedikation mit Thrombolytika,
• das Auftreten von früher Reokklusion und später Restenosierung nach PTCA,
• die thrombinabhängige Proliferation von Glattmuskelzellen,
• die Akkumulation aktiven Thrombins im ZNS,
• das Tumorwachstum sowie gegen die Adhäsion und Metastasierung von Tumorzellen.

4. Verbindungen der Formel I gemäß Anspruch 1 zur Beschichtung von Oberflächen.

5. Verwendung der Verbindungen der Formel I gemäß Anspruch 1 zur Herstellung von Arzneimitteln gegen:
• Krankheiten, deren Pathomechanismus direkt oder indirekt auf der proteolytischen Wirkung von Kininogenasen, insbesondere Kallikrein beruht,
• Entzündungskrankheiten wie Asthma, Pankreatitis, Rhinitis, Arthritis, Urticaria und anderen inneren Krankheiten, bei denen Kallikrein eine Rolle spielt.

6. Verbindungen der Formel 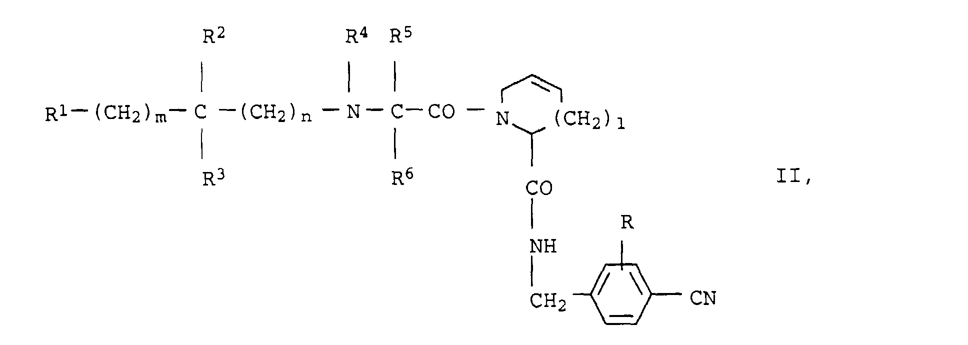 worin die Reste R, R¹, R², R³, R⁴, R⁵ und R⁶ sowie l, m und n die in Anspruch 1 genannten Bedeutungen besitzen.

7. Verbindungen der Formel 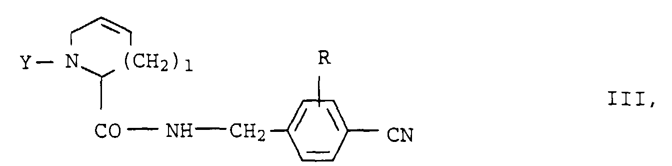 worin l und R die Bedeutung gemäß Anspruch 1 besitzt und Y eine N-Schutzgruppe, eine N-terminal geschützte oder ungeschützte Aminosäure oder H- bedeutet.

8. Inhibitor von Serinproteasen enthaltend ein strukturelles Fragment der Formel 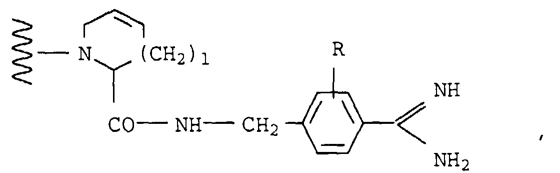 worin l = 0 oder 1 und R = H- oder C₁₋₄-Alkyl- bedeutet.

9. Verwendung des strukturellen Fragments der Formel 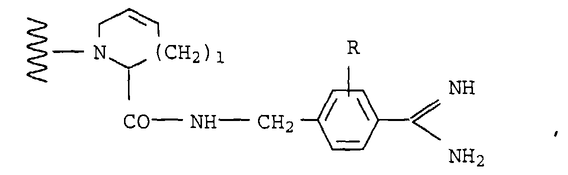 worin l = 0 oder 1 und R = H- oder C₁₋₄-Alkyl- bedeutet,
als Bestandteil der Gesamtstruktur von Verbindungen mit Serinproteasen inhibierender Wirkung.

## Claims

1. A compound of the formula I 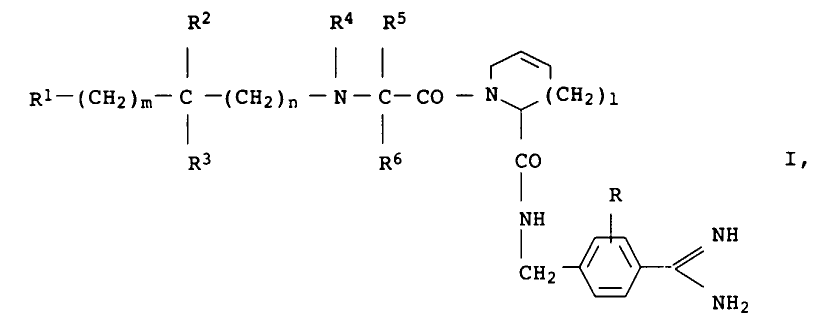 where R, R¹, R², R³, R⁴, R⁵ and R⁶, and l, m and n have the following meanings:
l 0 or 1,
m 0, 1 or 2,
n 0, 1 or 2,
R H or C₁₋₄-alkyl-,
R¹ HOOC-, C₁₋₆-alkyl-OOC-, benzyl-OOC- or -OH,
R² H-, C₁₋₄-alkyl- or R¹-(CH₂)ₘ-,
R³ H- or C₁₋₄-alkyl- which can be substituted by -OH or -COOH,
R⁴ H-, C₁₋₄-alkyl-, HOOC-C₁₋₄-alkylene-,
R⁵ C₁₋₈-alkyl-, cycloalkyl-(CR⁸R⁹)ᵣ-, (r = 0 or 1, R⁸, R⁹ = H-, cycloalkyl- or C₁₋₄-alkyl-), in which up to four CH₂ groups in the cycloalkyl radical can be replaced, independently of one another, by CR¹⁰R¹¹ (R¹⁰ = H- or C₁₋₄-alkyl-, R¹¹ = C₁₋₄-alkyl-) and/or the CH group in the cycloalkyl radical which is bonded CR⁸R⁹ can be replaced by CR¹² (R¹² = C₁₋₄-alkyl-), and/or one or two C-C single bond(s) in the ring can be replaced by a C=C double bond,
R⁶ H-, C₁₋₄-alkyl- or
R⁴ and R⁵ together -CH₂-CH₂-CH(R⁷)-, (R⁷ = H-, phenyl- or cyclohexyl-)
R² and R⁵ together -CH₂-CH₂- or -CH₂-CH₂-CH₂-, in which one hydrogen atom can be replaced by C₁₋₄-alkyl-, phenyl- or cycloalkyl-,
and salts thereof with physiologically tolerated acids.

2. A compound of the formula I as claimed in claim 1 for use for controlling diseases.

3. The use of a compound of the formula I as claimed in claim 1 for producing drugs for:
• diseases whose pathogenetic mechanism derives directly or indirectly from the proteolytic effect of thrombin,
• diseases whose pathogenetic mechanism derives from thrombin-dependent activation of receptors and signal transduction,
• diseases associated with stimulation or inhibition of the expression of genes in body cells,
• diseases deriving from the mitogenic effect of thrombin,
• diseases deriving from the thrombin-dependent change in the contractility and permeability of epithelial cells,
• thrombin-dependent thromboembolic events,
• disseminated intravascular coagulation,
• reocclusion and for reducing the reperfusion time on comedication with thrombolytics,
• the occurrence of early reocclusion and late restenosis after PTCA,
• the thrombin-dependent proliferation of smooth muscle cells,
• the accumulation of active thrombin in the CNS,
• tumor growth, and to prevent adhesion and metastasis of tumor cells.

4. A compound of the formula I as claimed in claim 1 for coating surfaces.

5. The use of a compound of the formula I as claimed in claim 1 for producing drugs for:
• diseases whose pathogenetic mechanism derives directly or indirectly from the proteolytic effect of kininogenases, especially kallikrein,
• inflammatory diseases such as asthma, pancreatitis, rhinitis, arthritis, urticaria and other internal diseases in which kallikrein is involved.

6. A compound of the formula 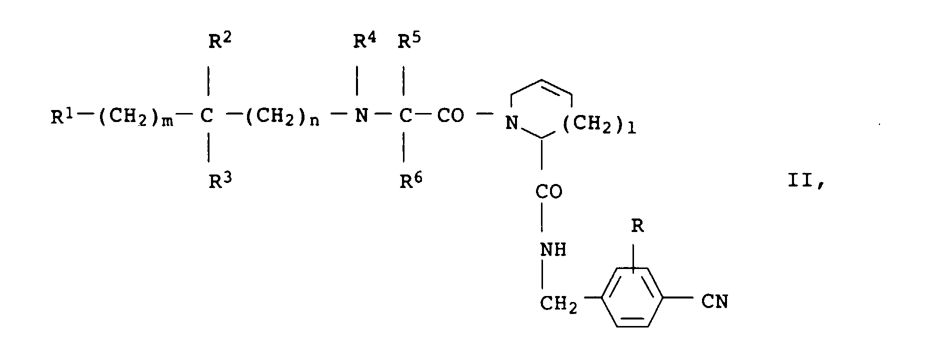 where R, R¹, R², R³, R⁴, R⁵ and R⁶, and l, m and n have the meanings stated in claim 1.

7. A compound of the formula 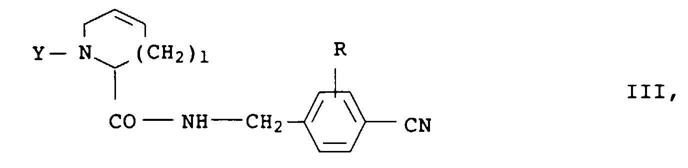 where l and R have the meanings set forth in claim 1, and Y is an N protective group, an N-terminally protected or unprotected amino acid or H-.

8. A serine protease inhibitor comprising a structural fragment of the formula 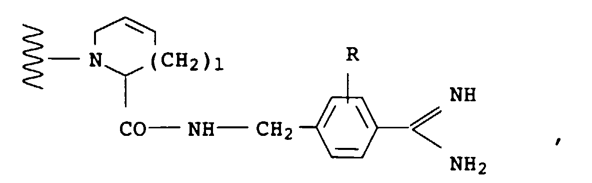 where l is 0 or 1 and R is H- or C₁₋₄-alkyl-.

9. The use of the structural fragment of the formula 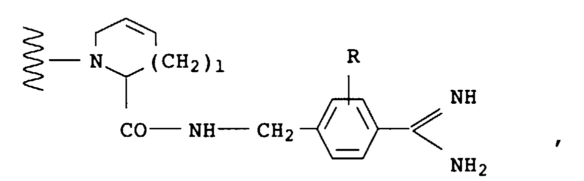 where l is 0 or 1 and R is H- or C₁₋₄-alkyl-,
as a constituent of the overall structure of compounds having a serine protease inhibiting action.

## Revendications

1. Composés de formule I 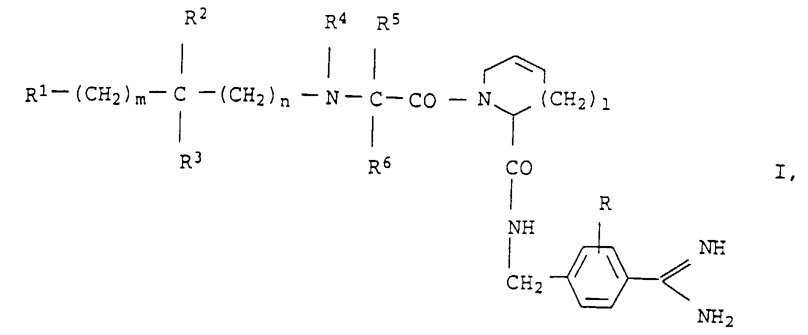 dans laquelle les symboles R, R¹, R², R³, R⁴, R⁵ et R⁶ et les indices l, m et n ont les significations suivantes :
l : 0 ou 1,
m : 0, 1 ou 2,
n : 0, 1 ou 2,
R : H ou alkyle en C1-C4,
R¹ : HOOC-, (alkyle en C1-C6)-OOC-, benzyl-OOC- ou -OH,
R² : H, alkyle en C1-C4 ou R¹-(CH₂)ₘ-,
R³ : H ou alkyle en C1-C4 qui peut être substitué par -OH ou -COOH,
R⁴ : H, alkyle en C1-C4, HOOC-(alkylène en C1-C4)-,
R⁵ : alkyle en C1-C8, cycloalkyl-(CR⁸R⁹)r- (r = 0 ou 1, R⁸,
R⁹ = H, cycloalkyle ou alkyle en C1-C4), jusqu'à quatre motifs CH₂ du groupe cycloalkyle pouvant être remplacés, indépendamment les uns des autres, par CR¹⁰R¹¹ (R¹⁰ = H ou alkyle en C1-C4, R¹¹ = alkyle en C1-C4) et/ou le motif CH du groupe cyclyoalkyle relié à CR⁸R⁹ pouvant être remplacé par CR¹² (R¹² = alkyle en C1-C4) et/ou une ou deux liaisons simples C=C du cycle pouvant être remplacées par une double liaison C=C,
R⁶ : H, alkyle en C1-C4, ou bien
R⁴ et R⁵, ensemble : -CH₂-CH₂-CH(R⁷)- (R⁷ = H, pényle ou cyclohexyle),
R² et R⁵, ensemble : -CH₂-CH₂- ou -CH₂-CH₂-CH₂-, un atome d'hydrogène pouvant être remplacé par un groupe alkyle en C1-C4, phényle ou cycloalkyle,
et leurs sels d'acides acceptables pour l'usage pharmaceutique.

2. Composés de formule I de la revendication 1 pour l'utilisation dans le traitement de maladies.

3. Utilisation des composés de formule I de la revendication 1 pour la préparation de médicaments contre :
- les maladies dont le pathomécanisme est en relation directe ou indirecte avec l'activité protéolytique de la thrombine,
- les maladies dont le pathomécanisme est en relation avec l'activation, fonction de la thrombine, de récepteurs et à des transductions de signaux,
- les maladies qui s'accompagnent d'une stimultation ou d'une inhivition des expressions géniques dans les cellules corporelles,
- les maladies en relation avec l'activité mitogène de la thrombine,
- les maladies dues à une modification, en relation avec la thrombine, de la contractilité et de la perméabilité des cellules épithéliales,
- les accidents thrombo-emboliques en relation avec la thrombine,
- une coagulation intravasculaire disséminée,
- une réocclusion et pour diminuer le temps de reperfusion dans une comédication avec des thrombolytiques,
- l'apparition d'une réocclusion prématurée et ultérieurement d'une resténose après PTCA,
- la prolifération, due à la trhombine, de cellules des muscles lisses,
- l'accumulation de thrombine active dans le ZNS,
- la croissance de tumeurs et contre l'adhérence et la métastase de cellules tumorales.

4. Composés de formule I de la revendication 1 pour le revêtement de surfaces.

5. Utilisation des composés de formule I selon la revendication 1 pour la préparation de médicaments contre :
- les maladies dont le pathomécanisme est en relation directe ou indirecte avec l'activité protéolytique de kininogénases, en particulier de la kallikréine,
- les maladies inflammatoires telles que l'asthme, la pancréatite, la rhinite, l'arthrite, l'urticaire et d'autres maladies internes dans lesquelles la kallikréine intervient.

6. Composés de formule 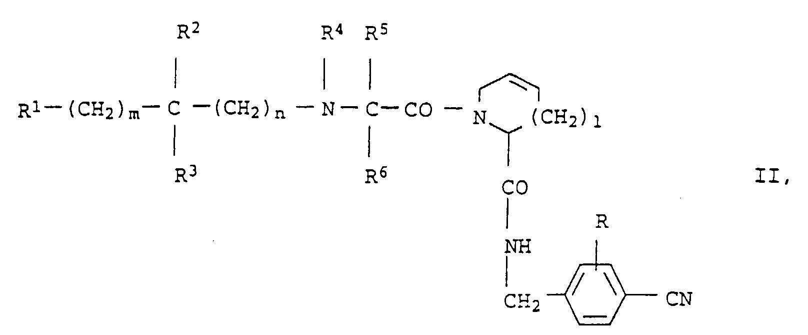 dans laquelle les symboles R, R¹, R², R³, R⁴, R⁵ et R⁶ et les indices 1, m et n ont les significations indiquées dans la revendication 1.

7. Composés de formule 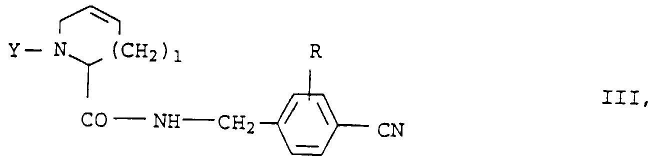 dans laquelle l et R ont les significations indiquées dans la revendication 1 et Y représente un groupe protecteur de l'azote, un aminoacide protégé ou non protégé sur l'azote terminal, ou H.

8. Inhibiteur des sérine-protéases contenant un fragment structurel de formule 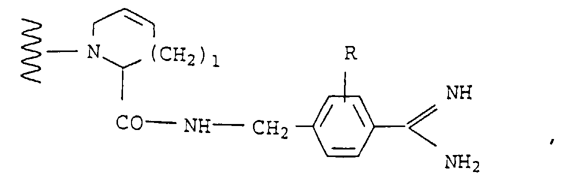 dans laquelle 1 = 0 ou 1 et R = H ou alkyle en C1-C4.

9. Utilisation du fragment structurel de formule 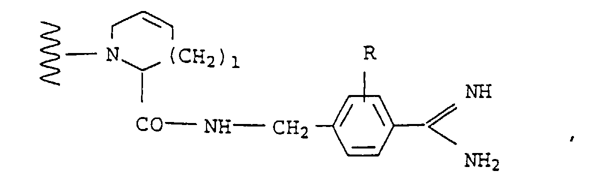 dans laquelle l = 0 ou 1 et R = H ou alkyle en C1-C4, en tant que constituant de la structure globale de composés ayant une activité inhibitrice sur les sérine-protéases.
